# EUROPEAN PATENT APPLICATION

(11) **EP 3 550 486 A1**
(43) Date of publication of application: **09.10.2019**
(21) Application number: 18166180.2
(22) Date of filing: 06.04.2018
(51) Int. Cl.: G06Q 10/08, G06Q 50/28, B08B 13/00, G05D 1/02

(54) **SYSTEM FOR WASHING AND INSPECTING MEDICAL EQUIPMENT**

(71) Applicant: Gibotech A/S, 5220 Odense SØ (DK)
(72) Inventor: Vinge, Lars, 5220 Odense SØ (DK)
(74) Representative: AWA Denmark A/S

(57) **Abstract**

The invention relates to a system for washing and inspecting medical equipment comprising a washing station for washing and inspecting medical equipment. The washing station comprises a first working position for a human operator, a transport vehicle for transporting medical equipment to the washing station, and a control unit. The control unit is configured to receive first user information related to the human operator working at the first working position, and, in response thereto, to control the system based on the first user information.

## Description

### FIELD

The invention relates to a system for washing and inspecting medical equipment, to a method for controlling a system for washing and inspecting medical equipment, a computer readable medium to implement a method for controlling a system for washing and inspecting medical equipment, and to a use of the system for washing and inspecting medical equipment.

### BACKGROUND

Systems for washing and inspecting used medical equipment are widely known in hospital environments.
Used medical equipment in hospitals, clinics or the like have to be washed and inspected after a medical procedure before they can be used again.
The washing and inspection of medical equipment such as surgical equipment is typically done in a centralized system or facility within a hospital. Before being washed, sterilized and packed to be reused, the medical equipment is typically manually pre-washed/ roughly washed and inspected by a staff member.

These systems usually comprise a washing table with a working position for a human operator, the used dirty medical equipment is placed in trays or containers, and transported with trolleys to the washing table, to be washed and inspected.

However, many of the steps/processes in these systems are rather repetitive and inefficient. Staff members are monitoring or performing many of the processes manually, making them time- and labor-consuming.
Another problem arising is that many of the processes may stand still for a period of time whereby the stations are in standby.

A solution to that may be to automate some processes performed in the washing and inspection stations, whereby the efficiency of such systems may be improved.

An example of a system for washing and inspecting medical equipment where automated processes are implemented is disclosed in WO2017077073. The station comprises a dirty area and a clean area, a washing table with two washing sides, washers, and an unmanned vehicle for loading, unloading, and transporting of medical equipment in baskets, where the unmanned vehicle can adjust in the vertical and horizontal direction. The station further comprises control devices for communication with the unmanned vehicles. The unmanned vehicles can be monitored to travel to specific pick-up points at the washing tables.

Nevertheless, many of the processes involved in the washing and inspection of medical equipment can still be improved in the above system.

Thus, it remains a problem to provide a more efficient system for washing and inspecting medical equipment.

### SUMMARY

According to a first aspect, the invention relates to a system for washing and inspecting medical equipment comprising:
- a washing station for washing and inspecting medical equipment, the washing station comprising a first working position for a human operator
- a transport vehicle for transporting medical equipment to the washing station,
- a control unit
wherein the control unit is configured to receive first user information related to the human operator working at the first working position and in response thereto to control the system based on the first user information.

By having a control unit configured to control the system based on first user information, the system may function in a more efficient and automated way.

The medical equipment may be stored in a centralized storage before being transported to the system for washing and inspection. The medical equipment may be sorted at the storage. The storage may be controlled by the system. The medical equipment may be transported from the storage to the system by a transport vehicle of the system. The medical equipment may be any type of equipment used in relation with a hospital or clinic, that prior to be reused need to be washed and inspected e.g. surgical instruments. The medical equiment may be transported to the system in a container. The medical equipment may be sorted before being put in a container.

The washing station may comprise e.g. a table, a sink, a washing and flushing arrangement, an inspection arrangement as a magnifying glass, a blowing or air gun, a water gun, and/or a trash receptacle.
The table of the washing station may be height adjustable. The control unit may control the height of the table based on the first user information.
The transport vehicle may be an automated or semi-automated vehicle. The transport vehicle may comprise a drive mechanism and travelling means such as wheels or chain-wheels. The transport vehicle may comprise navigation means such as sensors and/or cameras for navigation in order to orientate itself and such that it doesn't collide with any other object. The transport vehicle may be guided with guiding means such as floor stripes e.g. colored stripes or magnetic stripes. The guiding means may also be vision, or laser means.
The transport vehicle may follow markers or wires in the floor, lasers or any other guiding means for navigation. The transport vehicle may also be fully configured to travel without any guiding means e.g. by having a preprogrammed travelling path or by having a map of the navigation area for determining the travelling path based on the map and the navigation means. The transport vehicle may comprise a motorized roller, belt, or chain conveyor for delivering medical equipment.

The control unit may be operatively connected to the different elements of the system, e.g. the transport vehicle, the washing station, or any other element of the system. The control unit may be wirelessly connected to the different elements of the system. Furthermore, the control unit may be connected to an external network, where the network may e.g. be a network within a hospital or a global network such as the internet.
The control unit may be configured to receive an input e.g. the first user information. The control unit may be further configured to send an output e.g. a control signal to the different elements of the system. The first user information may be data pre-stored on a data storage unit. Alternatively the first user information may be received at the control unit e.g. from a sensor or a camera.
The control unit may have access to the storage database to access the first user information.

In some embodiments the system comprises a user input unit operatively connectable to the control unit, and the control unit is configured to receive control signals from the user input unit and control said system based on said control signals.

A human operator working at the first working position may indicate at the user input unit when he/she has finished washing and inspecting medical equipment. Further a human operator may indicate at the user input unit when he/she is ready to receive medical equipment to be washed and inspected.
The user input unit may e.g. have a keyboard, a touch screen or the like, whereby the human operator at the first working position may receive instructions, give instructions, and/or provide feedback to the system.

In some embodiments the control unit has access to a plurality of user profiles for human operators of the system, and the control unit is configured to receive first user information related to the human operator working at the first working position by receiving a user profile related to said human operator working at the first working position.

The control unit may therefore control the system based on the user profile of the human operator working at the first working position. The control unit may thereby control the system in an efficient and automatic way e.g. by transporting medical equipment matching to a user profile or by adjusting the height of the table based on the user profile.
A user profile for a human operator may comprise information e.g. the height, the age, the gender, the position, the experience, and/or the medical equipment preferences of the human operator. A user profile of a human operator may be updated with new information related to the human operator. The update of a user profile may be performed automatically by the control unit or manually by a human operator.
The human operator may e.g. login at the user input unit when starting to work at the first working position and logout when finishing to work.

In some embodiments the system is configured to wash and inspect a plurality of different classes of medical equipment, each class requires a specific user authorization to inspect and wash medical equipment of said class, each of the plurality of user profiles specifies the user authorizations of the respective human operator, wherein the control unit is configured to control the system so that the transport vehicle only transports to the washing station medical equipment of classes which the human operator working at the first working position has user authorization for.

By only transporting to the washing station medical equipment of classes that the human operator working at the first working position has user authorization for, the human operator does not have to assess or decide which medical equipment he/she has user authorization to wash and inspect. The system may therefore be further automated, and be able to function more independently. Furthermore, the system may not be dependent on an order or a monitoring from a human operator or a staff member, to choose or transport medical equipment to be washed and inspected by a human operator working at the first working position. Additionally, errors in the washing and inspection due to human operators lacking user authorization for washing and inspecting certain classes of medical equipment may be avoided. This may provide a better quality for the washing and inspection.

A class of medical equipment may comprise or consist of different types of medical equipment e.g. medical equipment of different sizes or medical equipment of substantially the same size but having other different characteristics. A class of medical equipment may consist of a specific type of medical equipment e.g. surgical equipment of a specific kind, such as cutters, retractors, or graspers. Different characteristics may be medical equipment of a different kind.
As an example, a class of medical equipment may comprise or consist of substantially small medical equipment of mixed kind, but may for example also comprise or consist of surgical equipment which may have both substantially small and substantially big equipment. The control unit may be configured to control the system such that the transport vehicle transports medical equipment of different classes to the washing station, as long as the human operator working at the first working position has user authorization to wash and inspect the medical equipment.
In some embodiments each user profile of the plurality of user profiles comprises information about working preferences of the human operators.
In some embodiments the working preferences comprise one or more items selected from the list comprising:
working height preferences and/or medical equipment preferences.

By having information about the working preferences of the human operators, the control unit may be configured to control the system such that the working station may be adjusted based on the working preferences of the human operator working at the first working position. Further, the control unit may control the system such that the transport vehicle transports medical equipment based on the medical equipment preferences of the human operator working at the first working position to the washing station. The washing station may be pre-adjusted for a human operator and when a new human operator is to work at the washing station the control unit may change the adjustment of the washing station and the medical equipment transported by the transport vehicle to the washing station in an automatic way. The system may therefore be more efficient and function in a more automatic way without having to perform manual adjustment and/or receive orders from a human operator.

In some embodiments the system further comprises a receiving element arranged in connection with the washing station, the receiving element being configured to receive a container comprising medical equipment transported by the transport vehicle to be washed and inspected, the receiving element being adjustable in height and/or horizontal position, and wherein the control unit is further configured to control the receiving element.
In some embodiments the receiving element is movable between a receiving position at a first height and a working position at a second height, said working position is adjustable in height, and wherein the receiving element is configured to receive a container at the receiving position and subsequently move to the working position.
By having a receiving element in connection with the washing station, the receiving element being adjustable in height position, the receiving element may be adjusted to the height of the transport vehicle. The height of the transport vehicle may therefore be constant, whereby a simpler transport vehicle may be provided. The receiving element may comprise a motorized roller, belt, or chain conveyor for receiving a container with medical equipment from a transport vehicle and/or for delivering an empty container to a transport vehicle. A container may be transferred e.g. by the conveyor of the transport vehicle, by the conveyor of the receiving element, or by the combination of the conveyor of the transport vehicle and the conveyor of the receiving element.
The receiving element may be able to communicate with the washing station and the transport vehicle independently from the control unit. This communication may be done wirelessly.
The receiving positon may be at a constant height i.e. if the transport vehicle has a constant height.
The receiving element may also deliver a container to a transport vehicle when the container is empty.

The receiving element may further be movable in the horizontal direction e.g. to compensate for a spacing between the receiving element and the transport vehicle.
In some embodiments the receiving element has a first and a second area, where the first area is configured to receive a container with medical equipment transported by the transport vehicle to be washed and inspected and the second area is configured to deliver an empty container to the transport vehicle.

The containers comprising medical equipment may be of different kind e.g. have different sizes i.e. depending on the classes of medical equipment or the type of medical equipment. The containers used with the receiving element and the containers used with the outgoing element may be different containers.

In some embodiments the system further comprises an outgoing element arranged in connection with the washing station, the outgoing element being configured to deliver a container comprising medical equipment that have been washed and inspected, to the transport vehicle, the outgoing element being adjustable in height, and wherein the control unit is further configured to control the outgoing element, the outgoing element being movable between a working position at a first height and an outgoing position at a second height, said working position being adjustable in height, and wherein the outgoing element is configured to be at the working position while the human operator is washing and inspecting the medical equipment, and subsequently move to the outgoing position to deliver the container to the transport vehicle.

By having an outgoing element in connection with the washing station, the outgoing element being adjustable in height, the outgoing element may be adjusted to the height of the transport vehicle. The height of the transport vehicle may therefore be constant, whereby a simpler transport vehicle may be provided. The outgoing element may comprise a roller, belt, or chain conveyor for delivering a container with washed and inspected medical equipment to a transport vehicle and/or for receiving an empty container from a transport vehicle. A container may be transferred by the conveyor of the outgoing element, the conveyor of the transport vehicle or by the combination of the conveyor of the transport vehicle and the conveyor of the outgoing element.
The outgoing element may be able to communicate with the washing station and the transport vehicle independently from the control unit. This communication may be done wirelessly.
The outgoing positon may be at a constant height i.e. if the transport vehicle has a constant height.
The outgoing element may also deliver a container to a transport vehicle when the container is empty.
The outgoing element may further be movable in the horizontal direction e.g. to compensate for a spacing between the outgoing element and the transport vehicle.

The transport vehicles transporting to and from the receiving element and to and from the outgoing element may be different transport vehicles e.g. dirty transport vehicles for the receiving element and clean transport vehicles for the outgoing element.
In some embodiments the working position of the receiving element and/or outgoing element is selected by the control unit based on the first user information.
The working position may e.g. be determined based on the, user profiles, the working preferences, the class of medical equipment, and/or the medical equipment in a container. The working position of the receiving element and the outgoing element may be the same but may also be different. By having the working position selected by the control unit based on the first user information, the human operator working at the first working position may be put in the most optimal conditions to perform the washing and inspection of medical equipment, whereby the efficiency of the system may be improved.

In some embodiments the control unit is configured to control the washing station, the transport vehicle, the receiving element, and/or the outgoing element based on the first user information.

By having a receiving element with a first and a second area, the transport vehicle may transport to the washing station, the next container with medical equipment to be washed and inspected before the previous container has been emptied. The human operator may therefore when having emptied a container with medical equipment to be washed and inspected, immediately pick from the next container with medical equipment to be washed and inspected without having to wait on the transport vehicle removing the empty container and delivering the next container. The transport vehicle may thereby also remove the empty container while the human operator has started with the next container and so on. The first and second area may both be configured to receive and deliver containers. The first and the second area may be configured to transfer containers to each other if one of the areas is empty of containers. The first and the second area may both be configured to receive containers with medical equipment from the transport vehicle and may both be configured to deliver empty containers to the transport vehicle.
In some embodiments the control unit has access to first user information such as scheduling information of the human operators of said system, and wherein the control unit is configured to control the system so that the transport vehicle transports medical equipment to the washing station based on the scheduling information of the human operators.
The control unit may be configured to schedule the washing and inspection of a class or type of medical equipment based on the scheduling information of the human operators. For example, a human operator being the only one having user authorization for a class of medical equipment may be away or occupied one week, and in response thereto the control unit may prioritize to transport the class or type of medical equipment to that specific human operator before said week. The washing and inspection of medical equipment may therefore be optimized based on the scheduling information of the human operators of the system.
The scheduling information of the human operators may e.g. be calendars comprising information about the tasks, the working hours, the shifts and/or the vacations.
In some embodiments the first user information comprises data log information of events in the system, such as data log information related to the washing and the inspection.
In some embodiments the control unit is configured to control the system based on the data log information so that the transport vehicle transports medical equipment to the washing station based on the data log information of events in the system.
The data log information may e.g. be washing and inspection times of a class or a type of medical equipment or of the medical equipment in a container, by a human operator.
The control unit may e.g. be configured to optimize the washing and inspection times of the system, so that the transport vehicle transports a class or a type of medical equipment to the human operator having the fastest washing and inspection times for said class or type of medical equipment.
In some embodiments the transport vehicle is selected from the following types of vehicles:
an AGV (automated guided vehicle), AGC (automated guided cart), LGV (laser guided vehicle), an UGV (unmanned ground vehicle), and/or a drone.

According to a second aspect, the invention relates to a method for controlling a system for washing and inspecting medical equipment said system comprising a washing station for washing and inspecting medical equipment, said washing station comprising a first working position for a human operator, a transport vehicle for transporting medical equipment to said washing station, and a control unit configured to control said system,
the method comprising the steps of:
- receiving at the control unit first user information related to a human operator working at a first working position
- processing at the control unit, the first user information to create first instructions
- sending said first instructions from the control unit to one or more elements of the system, for controlling the one or more elements of the system

In some embodiments the control unit has access to a plurality of user profiles for human operators of said system, and the step of receiving at the control unit first user information related to a human operator working at a first working position further comprises the step of:
- receiving at the control unit a user profile related to the human operator working at the first working position

In some embodiments the system for washing and inspecting medical equipment is configured to wash and inspect a plurality of different classes of medical equipment, each class requiring a specific user authorization to inspect and wash medical equipment of said class, and each of the plurality of user profiles specifying the user authorizations of the respective human operator, and the step of processing at the control unit, the first user information to create first instructions further comprises the steps of:
- determining at the control unit a class/type of medical equipment that the human operator at the first working position has user authorization for based on the received user profile
- creating at the control unit the first instructions based on the previous step
   so that said transport vehicle only transports to the washing station medical equipment of classes which the human operator working at the first working position has user authorization for.

In some embodiments the user profiles comprise information about working preferences of the human operators, said working preferences comprising one or more items selected from the list comprising: working height preferences and/or medical equipment preferences, and wherein the first instructions are created based on the information about the working preferences of the human operator working at the first working position

In some embodiments the system further comprises a receiving element arranged in connection with the washing station, said receiving element being configured to receive a container comprising medical equipment transported by the transport vehicle to be washed and inspected, the receiving element being adjustable in height and/or horizontal position,
and the step of sending first instructions from the control unit to one or more elements of the system, for controlling the one or more elements of the system further comprises the step of:
- sending first instructions from the control unit to the receiving element for controlling said receiving element

In some embodiments the receiving element is movable between a receiving position at a first height and a working position at a second height, said receiving position being adjustable in height, and the receiving element is configured to receive a container at the receiving position and subsequently move to the working position, the first instructions specify the working position for the receiving element,
and the step of sending first instructions from the control unit to one or more elements of the system, for controlling the one or more elements of the system further comprises the step of:
- sending first instructions from the control unit to the receiving element

In some embodiments the system further comprises an outgoing element arranged in connection with the washing station, said outgoing element being configured to deliver a container comprising medical equipment that have been washed and inspected to the transport vehicle, the outgoing element being adjustable in height and/or horizontal position, and being movable between a working position at a first height and an outgoing position at a second height, said working position being adjustable in height, and the outgoing element is configured to be at the working position while the human operator is washing and inspecting the medical equipment, and subsequently move to the outgoing position to deliver a container to the transport vehicle, the first instructions specify the working position for the outgoing element,
and the step of sending first instructions from the control unit to one or more elements of the system, for controlling the one or more elements of the system further comprises the step of:
- sending first instructions from the control unit to the outgoing element

In some embodiments the step of sending first instructions from the control unit to one or more elements of the system, for controlling the one or more elements of the system further comprises the step of:
- sending instructions related to the working position of the receiving element and/or outgoing element from the control unit to the receiving element and/or the outgoing element

In some embodiments the step of sending first instructions from the control unit to one or more elements of the system, for controlling the one or more elements of the system further comprises the step of:
- sending the first instructions from the control unit to the washing station, the transport vehicle, the receiving element, and/or the outgoing element

In some embodiments the control unit has access to first user information, said first user information comprising scheduling information for one or more human operators of the system, and the step of processing at the control unit, the first user information to create first instructions further comprises the step of:
- creating at the control unit first instructions based on the scheduling information

In some embodiments the first user information comprises data log information of events in the system, related to the washing and the inspection of medical equipment, and the step of processing at the control unit, the first user information to create first instructions further comprises the step of:
- creating at the control unit first instructions based on the data log information
According to a third aspect, the invention relates to a computer readable medium comprising computer readable code, wherein the computer readable medium is configured to implement a method according to the second aspect of the invention.

According to a fourth aspect, the invention relates to a use of a system according to a first aspect of the invention.

The different aspects of the present invention can be implemented in different ways including as systems for washing and inspecting medical equipment, a method for controlling systems for washing and inspecting medical equipment and use of systems for washing and inspecting medical equipment described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### BRIEF DESCRIPTION OF THE DRAWINGS

The above and/or additional objects, features and advantages of the present invention will be further elucidated by the following illustrative and non-limiting detailed description of embodiments of the present invention, with reference to the appended drawings, wherein:
Fig. 1 shows a block diagram of a system for washing and inspecting medical equipment, according to an embodiment of the invention.
Fig. 2 shows a flow chart of a method for controlling a system for washing and inspecting medical equipment, according to an embodiment of the invention.
Fig. 3 shows a perspective view of a system for washing and inspecting medical equipment, according to an embodiment of the invention.
Fig. 4 shows a perspective view of a transport vehicle of a system for washing and inspecting medical equipment, according to an embodiment of the invention.
Fig. 5a and Fig. 5b show a perspective view of a receiving element of a system for washing and inspecting medical equipment, according to an embodiment of the invention, in respectively a retracted and a deployed state.
Fig. 6 shows a perspective view of an outgoing element of a system for washing and inspecting medical equipment, according to an embodiment of the invention.
Fig. 7 shows a top view of a system for washing and inspecting medical equipment, according to an embodiment of the invention.

### DETAILED DESCRIPTION

In the following description reference is made to the accompanying figures, which show by way of illustration how the invention may be practiced.

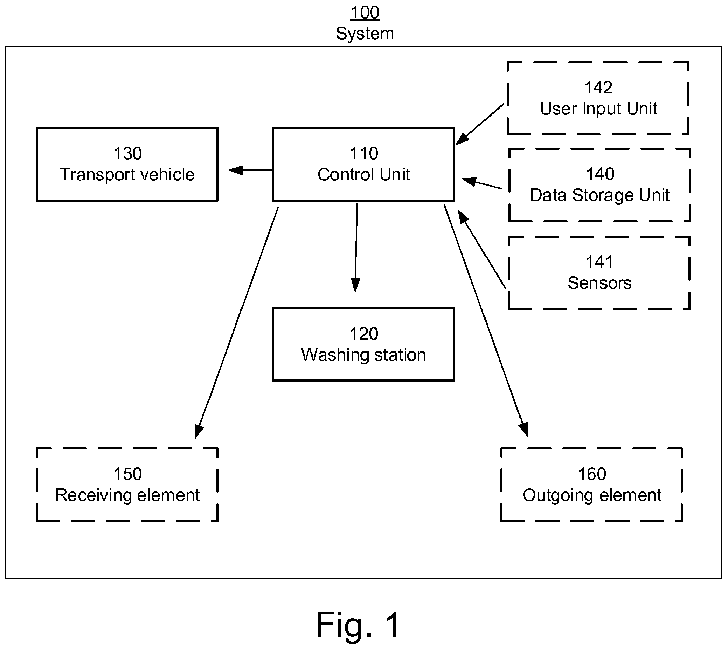

Fig. 1 shows a block diagram of a system 100 for washing and inspecting medical equipment, according to an embodiment of the present invention.
The system 100 comprises a washing station 120 for washing and inspecting medical equipment, where the washing station 120 comprises a first working position (not shown) for a human operator. The system 100 further comprises a transport vehicle 130 for transporting medical equipment to the washing station 120, and a control unit 110. The control unit 110 is configured to receive first user information related to the human operator working at the first working position, and, in response thereto, to control the system 100 based on the first user information.

Optionally, the control unit 110 has access to a plurality of user profiles for human operators of the system 100, and the control unit 110 is configured to receive first user information related to the human operator working at the first working position by receiving a user profile related to the human operator working at the first working position from a data storage unit 140.

Optionally, the control unit 110 is configured to receive first user information related to the human operator working at the first working position by receiving a signal from a sensor 141.

Optionally, the control unit 110 is configured to receive first user information related to the human operator working at the first working position by receiving a signal from a user input unit 142.

Optionally, the system 100 is configured to wash and inspect a plurality of different classes of medical equipment. Each class requires a specific user authorization to inspect and wash medical equipment of the class, and each of the plurality of user profiles specifies the user authorizations of the respective human operator. The control unit 110 is configured to control the system 100 so that the transport vehicle 130 only transports to the washing station 120 medical equipment of classes which the human operator working at the first working position has user authorization for.

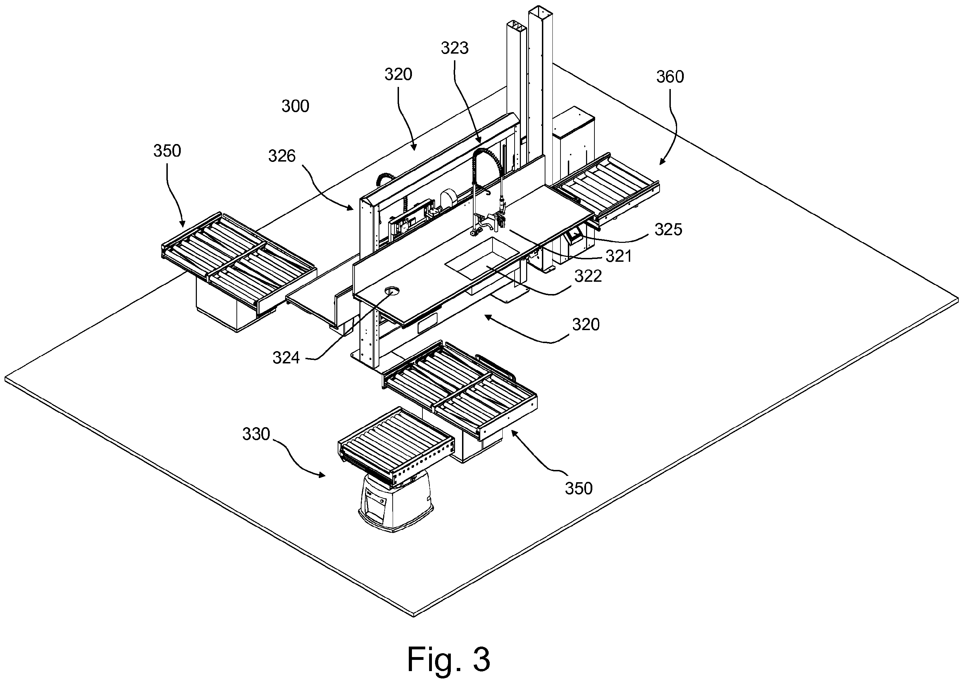

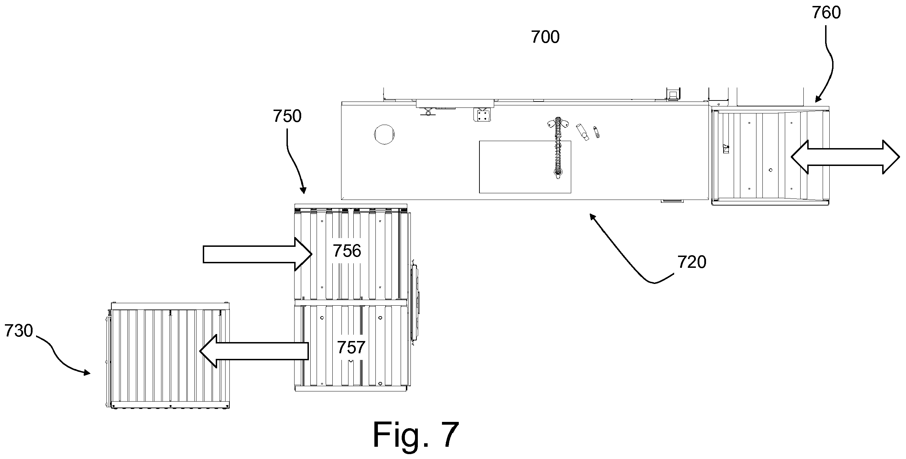

Optionally, the system 100 further comprises a receiving element 150 arranged in connection with the washing station 120 see also Fig. 3, Fig. 5a, Fig. 5b, and Fig. 7. The receiving element 150 is configured to receive a container comprising medical equipment transported by the transport vehicle 130 to be washed and inspected, see also Fig. 3 and Fig. 7. The receiving element 150 is adjustable in height and/or horizontal position. The control unit 110 is further configured to control the receiving element 150.

Optionally, the receiving element 150 is movable between a receiving position at a first height and a working position at a second height. The working position is adjustable in height, and the receiving element 150 is configured to receive a container at the receiving position and subsequently move to the working position, see also Fig. 3, Fig. 5a, Fig. 5b, and Fig. 7.

Optionally, the system 100 further comprises an outgoing element 160 arranged in connection with the washing station 120. The outgoing element 160 is configured to deliver a container comprising medical equipment that have been washed and inspected, to the transport vehicle 130. The outgoing element 160 is adjustable in height and/or horizontal position. The control unit 110 is configured to control the outgoing element 160. The outgoing element 160 is movable between a working position at a first height and an outgoing position at a second height. The working position is adjustable in height, and the outgoing element 160 is configured to be at the working position while the human operator is washing and inspecting the medical equipment, and subsequently move to the outgoing position to deliver the container to the transport vehicle 130.

Optionally, the working position of the receiving element 150 and the outgoing element 160 is selected by the control unit 110 based on the first user information, and the control unit 110 is configured to control the working position of the receiving element 150 and the outgoing element 160, see also Fig. 3, Fig. 6, and Fig. 7.

Optionally, the control unit 110 is configured to control the washing station 120, the transport vehicle 130, the receiving element 150, and the outgoing element 160 based on the first user information.

Optionally, the receiving element 150 has a first and a second area, where the first area is configured to receive a container with medical equipment transported by the transport vehicle 130 to be washed and inspected and the second area is configured to deliver an empty container to the transport vehicle 130, see also Fig. 3, Fig. 5a, Fig. 5b, and Fig. 7.

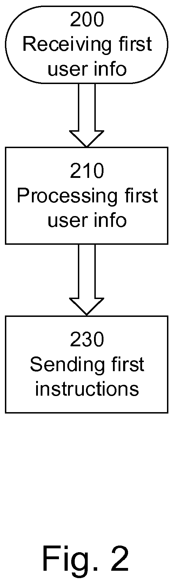

Fig. 2 shows a flow chart of a method for controlling a system for washing and inspecting medical equipment, according to an embodiment of the invention. The method comprises the steps of receiving 200 at the control unit first user information related to a human operator working at a first working position, 210 processing at the control unit, the first user information to create first instructions, and sending 230 the first instructions from the control unit to one or more elements of the system, for controlling the one or more elements of the system.

Fig. 3 shows a perspective view of a system 300 for washing and inspecting medical equipment, according to an embodiment of the invention.

The system 300 has in the embodiment shown on Fig. 3 two abutting washing stations 320 pointing away from each other. A receiving element 350 is in connection with each of the washing stations 320. The system 300 further has a transport vehicle 330, and an outgoing element 360 in connection with the washing station 320. The washing station 320 has a table 321 which is adjustable in height with a telescopic mechanism 326 arranged in connection with the table 321. The washing station 320 has a sink 322, a washing and flushing arrangement 323, a trash receptacle 324, and a user input unit 325 arranged at the table 321. The user input unit 325 is here shown in the form of a touchscreen.

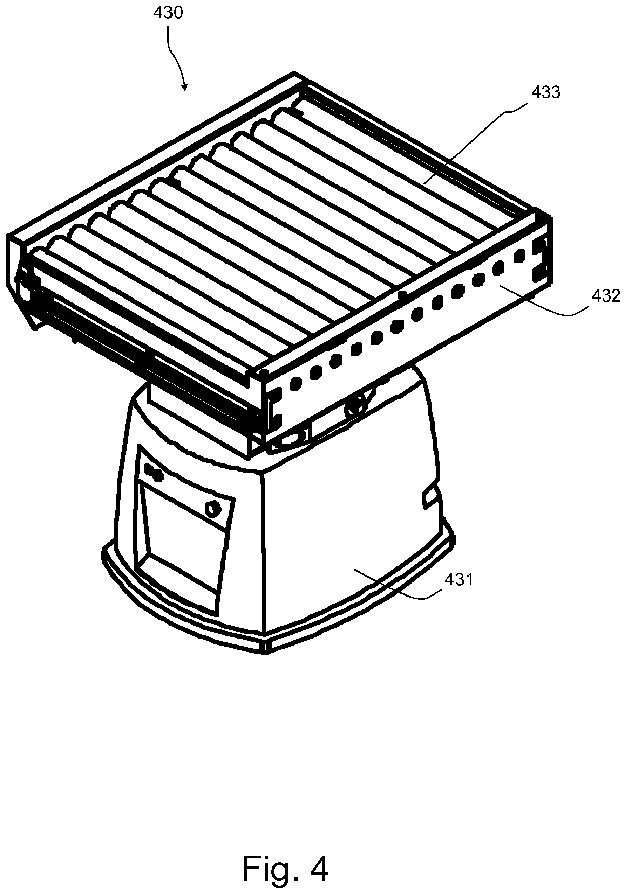

Fig. 4 shows a perspective view of a transport vehicle 430 of a system for washing and inspecting medical equipment, according to an embodiment of the invention. The transport vehicle 430 has a lower element 431 and an upper element 432. The lower element 431 has a drive mechanism (not shown) allowing the transport vehicle 430 to travel. The upper element 432 is arranged on top of the lower element 431. The upper element 432 has a motorized roller conveyor 433 for delivering and receiving medical equipment. The upper element 432 is arranged to support medical equipment.

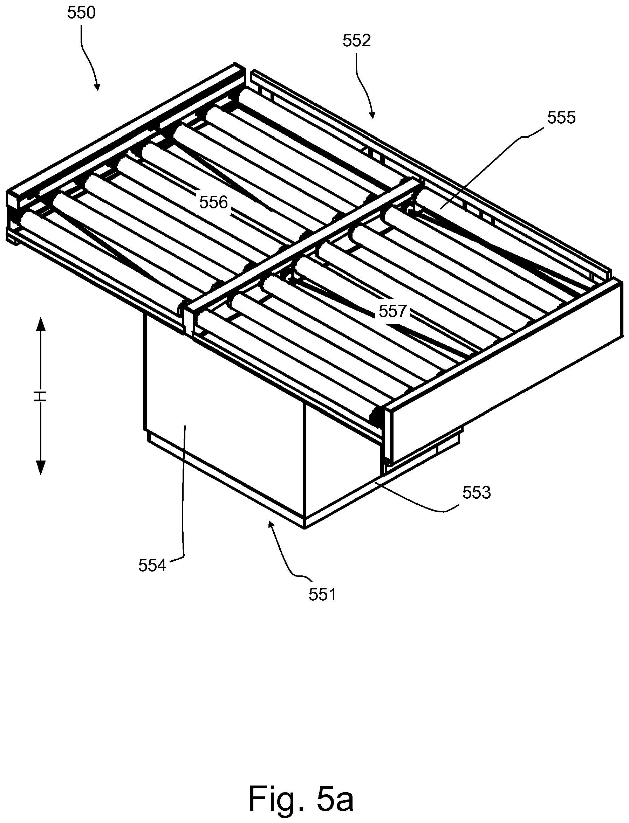

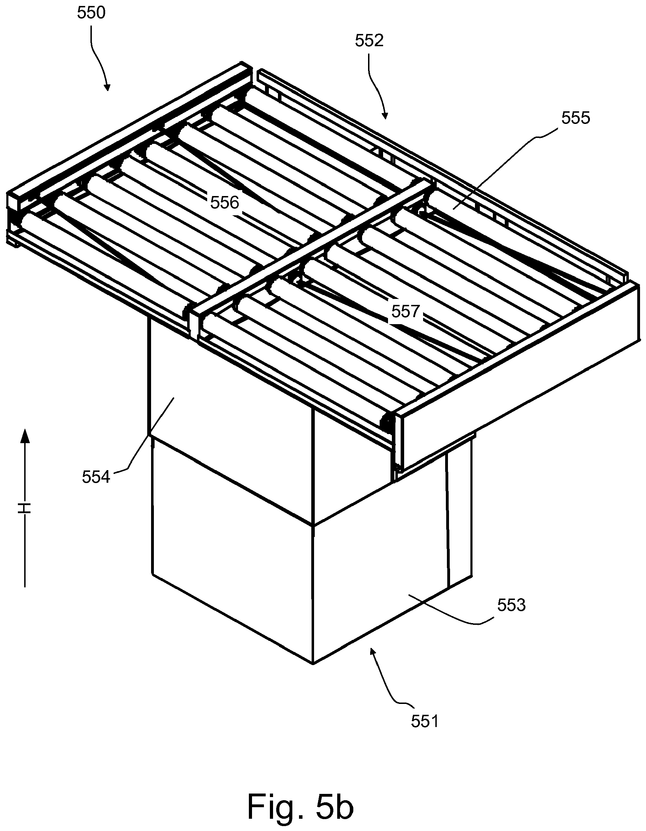

Fig. 5a and Fig. 5b show a perspective view of a receiving element 550 of a system for washing and inspecting medical equipment, according to an embodiment of the invention. Fig. 5a shows a receiving position of the receiving element 550 and Fig. 5b shows a working position of the receiving element 550. The working position may be selected by the control unit 110 based on first user information and adapted to e.g. the height of the human operator.
The receiving element 550 has a base element 551 and a top element 552 arranged on top of the base element 551. The base element 551 has a telescopic mechanism (not shown) to adjust the height of the top element 552, whereby the height may be adjusted to the height of the transport vehicle 130, 430, 730, the receiving position, or adjusted to the height of the washing station 120, 320, 720, the working position. The base element 551 has an inner portion 553 and an outer portion 554, where the inner portion 553 is arranged in the outer portion 554 in a slidable manner as shown in Fig. 5a and Fig. 5b. The outer portion 554 slides with respect to the inner portion 553 in the height direction H. Fig. 5a shows the base element 551 in a retracted state and Fig. 5b shows the base element 551 in a deployed state.
The top element 552 has a motorized roller conveyor 555 for delivering and receiving medical equipment. The top element 552 is arranged to support the medical equipment. The receiving element 550 has on the embodiment shown on Fig. 5a and Fig. 5b a first area 556 and a second area 557. Each of the first area 556 and the second area 557 have a roller conveyor 555. The receiving element 550 may thereby have two containers simultaneously, one on the first area 556 and one on the second area 557.

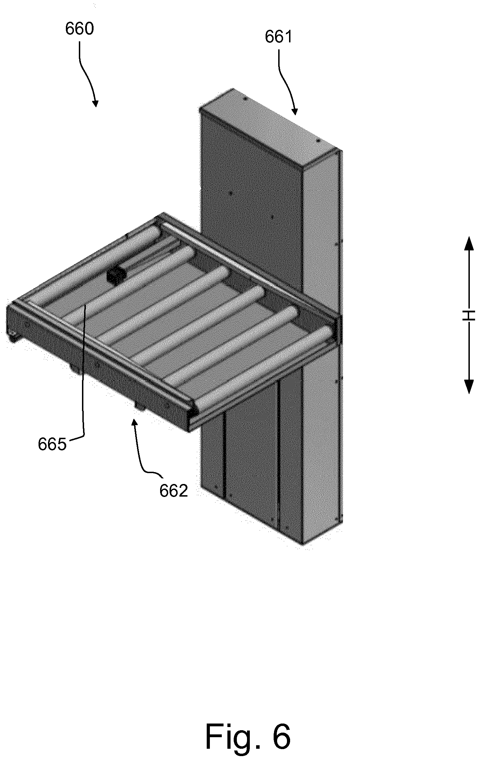

Fig. 6 shows a perspective view of an outgoing element 660 of a system for washing and inspecting medical equipment, according to an embodiment of the invention. The outgoing element 660 has a base element 661 and a surface element 662 arranged in connection with the base element 661. The base element 661 has a telescopic mechanism (not shown) to adjust the height of the surface element 662, whereby the height may be adjusted to the height of the transport vehicle 130, 430, 730 or adjusted to the height of the washing station 120, 320, 720. The surface element 662 is arranged to slide with respect to the base element 661 in the height direction H. The surface element 662 has a roller conveyor 665 for delivering and receiving medical equipment. The roller conveyor 665 may be a motorized roller conveyor 655. The surface element 662 is arranged to support medical equipment.

Fig. 7 shows a top view similar to the one of Fig. 3, of a system 700 for washing and inspecting medical equipment, according to an embodiment of the invention. The system 700 has a washing station 720. A receiving element 750 is arranged in connection with the washing station 720. The system 700 further has a transport vehicle 730, and an outgoing element 760 in connection with the washing station 720. The arrows on the figure at the receiving element show a sketch of a container with medical equipment being delivered from the transport vehicle 730 to the first area 756 of the receiving element 750 and simultaneously an empty container being delivered to the transport vehicle 730 from the second area 757 of the receiving element 750. The arrows at the outgoing element 760 show a sketch of a container with washed and inspected medical equipment being delivered from the outgoing element 760 to the transport vehicle 730 or an empty container being delivered from the transport vehicle 730 to the outgoing element 760.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A system for washing and inspecting medical equipment comprising:
• a washing station for washing and inspecting medical equipment, said washing station comprising a first working position for a human operator
• a transport vehicle for transporting medical equipment to said washing station,
• a control unit
wherein the control unit is configured to receive first user information related to the human operator working at the first working position, and, in response thereto, to control the system based on the first user information.

2. The system according to claim 1, wherein the control unit has access to a plurality of user profiles for human operators of said system, and wherein the control unit is configured to receive first user information related to the human operator working at the first working position by receiving a user profile related to said human operator working at the first working position.

3. The system according to claim 2, wherein the system is configured to wash and inspect a plurality of different classes of medical equipment, each class requires a specific user authorization to inspect and wash medical equipment of said class, each of the plurality of user profiles specifies the user authorizations of the respective human operator, wherein the control unit is configured to control the system so that the transport vehicle only transports to the washing station medical equipment of classes which the human operator working at the first working position has user authorization for.

4. The system according to claim 2 to 3, wherein each user profile of the plurality of user profiles comprises information about working preferences of the human operators.

5. The system according to claim 4, wherein the working preferences comprise one or more items selected from the list comprising:
working height preferences and/or medical equipment preferences.

6. The system according to claim 1-5, wherein the system further comprises a receiving element arranged in connection with the washing station, the receiving element being configured to receive a container comprising medical equipment transported by the transport vehicle to be washed and inspected, the receiving element being adjustable in height and/or horizontal position, and wherein the control unit is further configured to control the receiving element.

7. The system according to claim 6, wherein the receiving element is movable between a receiving position at a first height and a working position at a second height, said working position being adjustable in height, and wherein the receiving element is configured to receive a container at the receiving position and subsequently move to the working position.

8. The system according to claim 6-7, wherein the receiving element has a first and a second area, where the first area is configured to receive a container with medical equipment transported by the transport vehicle to be washed and inspected and the second area is configured to deliver an empty container to the transport vehicle.

9. The system according to claim 1-8, wherein the system further comprises an outgoing element arranged in connection with the washing station, the outgoing element being configured to deliver a container comprising medical equipment that have been washed and inspected, to the transport vehicle, the outgoing element being adjustable in height and/or horizontal position, and wherein the control unit is further configured to control the outgoing element, the outgoing element being movable between a working position at a first height and an outgoing position at a second height, said working position being adjustable in height, and wherein the outgoing element is configured to be at the working position while the human operator is washing and inspecting the medical equipment, and subsequently move to the outgoing position to deliver the container to the transport vehicle.

10. The system according to claim 7-9, wherein the working position of the receiving element and/or outgoing element is selected by the control unit based on the first user information.

11. The system according to any one of the preceding claims, wherein the control unit is configured to control the washing station, the transport vehicle, the receiving element, and/or the outgoing element based on the first user information.

12. The system according to any one of the preceding claims, wherein the control unit has access to scheduling information of the human operators of said system, and wherein the control unit is configured to control the system so that the transport vehicle transports medical equipment to the washing station based on the scheduling information of the human operators.

13. A method for controlling a system for washing and inspecting medical equipment said system comprising a washing station for washing and inspecting medical equipment, said washing station comprising a first working position for a human operator, a transport vehicle for transporting medical equipment to said washing station, and a control unit configured to control said system,
the method comprising the steps of:
• receiving at the control unit first user information related to a human operator working at a first working position
• processing at the control unit, the first user information to create first instructions
• sending said first instructions from the control unit to one or more elements of the system, for controlling the one or more elements of the system

14. A computer readable medium comprising computer readable code, wherein the computer readable medium is configured to implement the method according to claim 13.

15. Use of a system according to any of claim 1 to 12 for washing and inspecting medical equipment.
